Europäisches Patentamt

European Patent Office

Office européen des brevets

(11) Veröffentlichungsnummer: **0 317 954 B1**

## (12) EUROPÄISCHE PATENTSCHRIFT

(45) Veröffentlichungstag der Patentschrift: **28.10.92**

(21) Anmeldenummer: **88119412.0**

(22) Anmeldetag: **22.11.88**

(51) Int. Cl.⁵: **B26B 5/00**

(54) **Wechselklingenmesser.**

(30) Priorität: **26.11.87 DE 3740036**

(43) Veröffentlichungstag der Anmeldung:
**31.05.89 Patentblatt 89/22**

(45) Bekanntmachung des Hinweises auf die
Patenterteilung:
**28.10.92 Patentblatt 92/44**

(84) Benannte Vertragsstaaten:
**AT BE CH DE ES FR GB IT LI NL SE**

(56) Entgegenhaltungen:
**EP-A- 0 048 077**
**FR-A- 2 402 520**
**US-A- 1 754 650**
**US-A- 4 646 440**
**US-A- 4 660 287**

(73) Patentinhaber: **MARTOR-ARGENTAX E.H. Beermann KG**
**Heider Hof 60**
**W-5650 Solingen 1(DE)**

(72) Erfinder: **Beermann, Ewald Helmut**
**Lützowstrasse 238**
**W-5650 Solingen(DE)**

(74) Vertreter: **Ostriga, Harald et al**
**Stresemannstrasse 6-8 Postfach 20 13 27**
**W-5600 Wuppertal 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Messer mit einer Wechselklinge, die endseitig in einer Axialnut am Ende des Griffes zwischen einer Lasche und der Nutgrundfläche arretiert ist.

Ein derartiges Messer ist aus der EP-A-48 077 bekannt und wird unter anderem industriell zum Entgraten von Kunststoffteilen eingesetzt.

Zwar ist ein Klingenwechsel bei diesem bekannten Messer möglich, jedoch muß zu diesem Zweck das im Messergriff beweglich angeordnete, die Klinge führende Gleitstück in eine bestimmte Entriegelungsposition gebracht und sodann erst das Gleitstück einschließlich Klinge aus dem Messergriff entfernt werden.

Nachteilig ist daran einerseits, daß der Klingenwechsel nur relativ umständlich zu bewerkstelligen ist und andererseits das bekannte Messer kompliziert aufgebaut ist. Der komplizierte Aufbau des bekannten Messers führt darüber hinaus zu hohen Herstellungskosten.

Angesichts dieser Probleme liegt der Erfindung die Aufgabe zugrunde, unter Vermeidung der aufgezeigten Nachteile das Messer der eingangs genannten Art so auszugestalten, daß es einfach aufgebaut und preiswert herzustellen ist und daß sich ein Klingenwechsel rasch und einfach vollziehen läßt.

Gelöst wird diese Aufgabe gemäß der Erfindung durch die im Kennzeichenteil des Hauptanspruches angegebenen Merkmale, wobei hinsichtlich bevorzugter Ausgestaltungen des erfindungsgemäßen Messers auf die Merkmale der Unteransprüche verwiesen wird.

Gemäß der Erfindung ist die Lasche als Klemmlasche ausgebildet und gleitverschieblich in Längshinterschneidungen gehalten, die beidseitig längs der Nutgrundfläche ausgebildet sind, wobei die Nutgrundfläche zu ihrem von der Schneide abgewandten Ende hin ansteigend ausgebildet ist, so daß die Wechselklinge über eine dadurch gebildete Verklemmung arretiert ist. Hierdurch läßt sich die Klinge in einer überraschend einfachen, schnellen und sicheren Weise arretieren.

Durch die Ausgestaltung gemäß den kennzeichnenden Merkmalen wird die Klemmlasche bei ihrer Zurückführung näher an die Nutgrundfläche herangeführt und klemmt damit durch die entstehende Keilwirkung die Klinge fest ein. Der Klingenwechsel läßt sich rasch und problemlos ausführen. Bereits durch eine Vorverschiebung der Klemmlasche über eine relativ kurze Distanz wird die Klinge freigegeben und läßt sich gegen eine neue Klinge austauschen. Nach Rückführung der Klemmlasche um die gleiche Distanz sitzt die neue Klinge fest, ohne Zuhilfenahme eines Werkzeuges. Weitere Einzelteile, die verlorengehen könnten, sind nicht vorhanden.

Gemäß einer Ausführungsform des erfindungsgemäßen Messers weist die Klemmlasche eine quer zur Längsmittelachse des Messers ausgerichtete, der Nutgrundfläche zugewandte Schulter auf, an welcher sich das rückwärtige Ende der Klinge abstützt. Beim Einsetzen einer neuen Klinge wird diese zwischen Nutgrundfläche und Klemmlasche eingeschoben, bis sie an der Schulter der Klemmlasche anstößt. Durch Zurückziehen der Klemmlasche zusammen mit der Klinge wird letztere arretiert.

Gemäß einer bevorzugten Ausführungsform trägt die Klinge einen zu ihrem rückwärtigen Ende hin offenen Axialschlitz, in welchen ein entsprechender zungenförmiger, die Schulter bildender Vorsprung der Klemmlasche eingreift. Der positive Eingriff der Klemmlasche in das Profil der Klinge gewährleistet eine verbesserte Klingenführung gegenüber beim Schnitt auftretenden Querkräften. Das gabelförmige Ende der Klinge wird beim Einsetzen einer neuen Klinge auf den zungenförmigen Vorsprung aufgeschoben, bevor eine Verklemmung durch Zurückziehen der Klemmlasche ausgeführt wird.

Zweckmäßigerweise trägt die Klemmlasche auf ihrer Außenseite einen Betätigungsvorsprung. Hierdurch wird die Verschiebung der Klemmlasche vereinfacht. Über den Betätigungsvorsprung lassen sich etwa die Kräfte des Daumens in einfacher Weise auf die Klemmlasche übertragen, so daß sich ein festes Anziehen der Lasche und damit ein sicheres Verklemmen ermöglichen läßt.

Bevorzugt ist der Reibungsbeiwert zwischen der Klemmlasche und der Klinge größer als zwischen der Klinge und dem Material der Nutgrundfläche. Damit wird beim Zurückziehen der Klemmlasche zugleich die Klinge ohne Schlupf mitgenommen, so daß kein zusätzlicher Axialdruck auf die Klingenspitze auszuführen ist. Als zweckmäßiges und daher besonders bevorzugtes Material für die Klemmlasche hat sich Kunststoff erwiesen, während der Messergriff und damit die Nutgrundfläche aus Metall, wie etwa einem Aluminiumdruckguß, bestehen. Dieser Materialeinsatz gewährleistet, daß beim Zurückziehen der Klemmlasche die Klinge stets schlupffrei mitgezogen wird, während andererseits eine sichere Halterung in Klemmzustand gewährleistet ist.

Weitere Einzelheiten, Vorteile und Merkmale ergeben sich aus der nachfolgenden Beschreibung eines bevorzugten Ausführungsbeispieles des erfindungsgemäßen Messers unter Bezugnahme auf die beigefügten Zeichnungen. Dabei zeigt im einzelnen:

Fig. 1 die Draufsicht auf das Messer gemäß der Erfindung,

Fig. 2 einen Teilschnitt entlang der Längsmittelachse durch den Messergriff des in Fig. 1 dargestellten Messers,

Fig. 3 einen Schnitt durch den Messergriff entlang der Schnittlinie III-III der Fig. 2 in größerem Maßstab,

Fig. 4 eine Seitenansicht der Klemmlasche, ebenfalls in größerem Maßstab,

Fig. 5 die Draufsicht auf die Klemmlasche gemäß Fig. 4, in gleichem Maßstab,

Fig. 6 die Ansicht der Klemmlasche von unten, in gleichem Maßstab,

Fig. 7 einen Schnitt durch die Klemmlasche entlang der Schnittlinie VII-VII der Fig. 5, und

Fig. 8 die Draufsicht auf die Klinge.

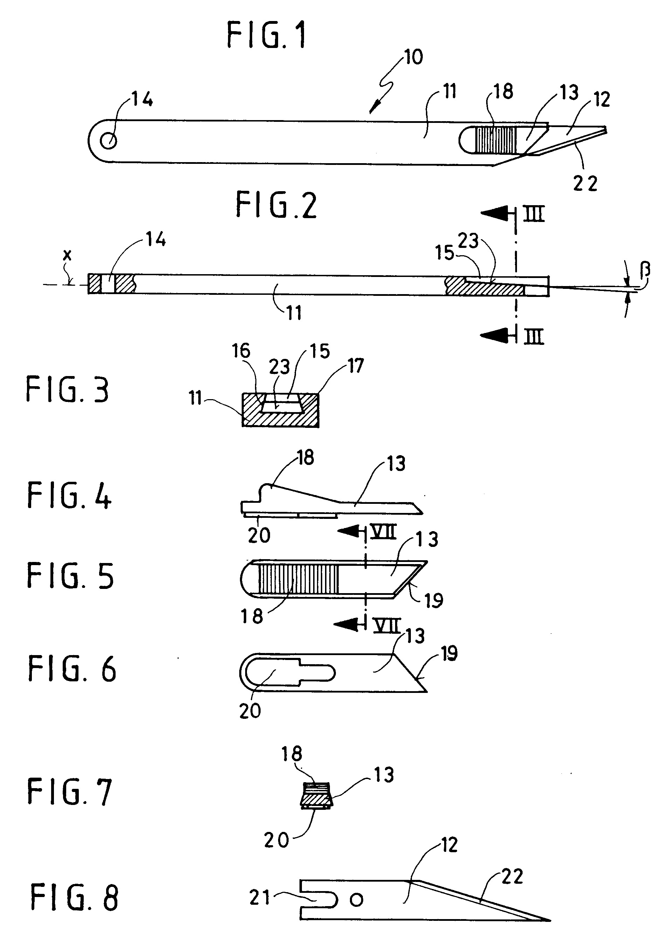

Das in Fig. 1 wiedergegebene Messer ist in seiner Gesamtheit mit der Bezugsziffer 10 versehen und umfaßt einen Griff 11, eine Klinge 12 sowie eine Klemmlasche 13. Der Griff 11 besteht aus Leichtmetall und trägt an seinem rückwärtigen Ende eine durchgehende Öffnung 14, die zum Aufhängen oder der Befestigung eines Haltefadens dient.

Am vorwärtigen Ende des Griffes 11 ist, von einer Seitenfläche ausgehend, eine Axialnut 15 eingebracht, deren Ausgestaltung sich im besonderen aus den Fig. 2 und 3 ergibt. Die Nutgrund fläche 23 bildet mit der Längsmittelachse des Griffes 11 einen Winkel $\beta$ dahingehend, daß sich die Nuttiefe zu ihrem rückwärtigen Ende hin verringert. Zur Verdeutlichung ist der Winkel $\beta$ in Fig. 2 etwas überhöht wiedergegeben.

Aus Fig. 3 läßt sich erkennen, daß die Nut 15 beiderseits durch eine Hinterschneidung 16 bzw. 17 begrenzt ist. Die Hinterschneidungen 16 und 17 dienen der seitlichen Führung der in den Fig. 4 bis 7 dargestellten Klemmlasche 13, deren Aufbau nachfolgend näher erläutert werden soll.

Die Klemmlasche 13 besitzt einen im wesentlichen trapezförmigen Querschnitt, wie im besonderen aus der Darstellung in Fig. 7 deutlich wird. Die seitlichen Schrägflächen greifen in die Hinterschneidungen 16 und 17 der Nut 15 ein und werden hierdurch längsverschieblich geführt. Auf der Oberseite trägt die Lasche 13 einen Betätigungsvorsprung 18, der materialeinheitlich mit der Lasche ausgebildet ist. Durch diesen Vorsprung 18 lassen sich die Kräfte für die Längsverschiebung der Klemmlasche 13 übertragen. Die vordere Kante ist mit einer Abschrägung 19 versehen, um der der Klinge 12 eine Führung über einen größeren Bereich zu geben. Auf der Unterseite trägt, wie Fig. 6 zeigt, die Lasche 13 einen zungenförmigen Vorsprung 20, der in einen rückwärtigen, endseitig offenen Axialschlitz 21 der Klinge 12 (Fig. 8) eingreift.

Die Klinge 12 trägt, gemäß der Darstellung in Fig. 8, eine zu ihrer Längsmittelachse schräg verlaufende Schneide 22. An ihrem rückwärtigen Ende ist der zuvor erwähnte endseitig offene Axialschlitz 21 für den Eingriff des zungenförmigen Vorsprunges 20 vorgesehen. Die Klinge 12 ist aus hochwertigem Federstahl hergestellt.

Der Klingenwechsel wird kurz wir folgt ausgeführt: Die Klemmlasche 13 wird durch die Kraftübertragung auf den Betätigungsvorsprung 18 um eine minimale Distanz nach vorne geschoben. Hierdurch wird die Klinge 12 aus ihrer Klemmposition freigegeben. Sie kann nach vorne herausgezogen und durch eine neue Klinge ersetzt werden. Nach dem Einschieben der Klinge 12 zwischen die Klemmlasche 13 und die Grundfläche 23 der Nut 15 wird die Klemmlasche 13 wieder zurückgezogen. Durch die Haftreibung zwischen Klemmlasche 13 und Klinge 12 wird letztere schlupffrei mit zurückgezogen. Durch Anziehen der Klemmlasche 13 in ihre rückwärtige Position wird die Klinge 12 in der Arbeitsposition arretiert. Rückzugs- und Arretierfunktion werden durch den Anstieg der Nutgrundfläche 23 gemäß dem Winkel $\beta$ bewirkt.

Es soll an dieser Stelle noch einmal ausdrücklich angeführt werden, daß es sich bei der vorangehenden Beschreibung lediglich um eine solche beispielhaften Charakters handelt, und daß verschiedene Modifikationen, im besonderen auch die Ausgestaltung der Schulter 20 betreffend, möglich sind, ohne dabei den Rahmen der Erfindung zu verlassen.

## Patentansprüche

1. Messer (10) mit einer Wechselklinge (12), die endseitig in einer Axialnut (15) am Ende des Griffes (11) zwischen einer Lasche (13) und der Nutfläche (23) arretiert ist, dadurch gekennzeichnet, daß die Lasche (13) als Klemmlasche (13) ausgebildet ist und gleitverschieblich in Längshinterschneidungen (16, 17) gehalten ist, die beidseitig längs der Nutgrundfläche (23) ausgebildet sind, wobei die Nutgrundfläche (23) zu ihrem von der Schneide (22) abgewandten Ende hin ansteigend ausgebildet ist, so daß die Wechselklinge (12) über eine dadurch gebildete Verklemmung arretiert ist.

2. Messer nach Anspruch 1, dadurch gekennzeichnet, daß die Klemmlasche (13) eine quer zur Längsmittelachse (x) des Messers (10) ausgerichtete, der Nutgrundfläche (23) zugewandte Schulter (20) aufweist, an welcher sich das rückwärtige Ende der Klinge (12) abstützt.

3. Messer nach einem der Ansprüche 1 oder 2, dadurch gekennzeichnet, daß die Klinge (12) einen zu ihrem rückwärtigen Ende hin offenen Axialschlitz (21) trägt, in welchen ein entsprechender, zungenförmiger, die Schulter bildender Vorsprung (20) der Klemmlasche (13) eingreift.

4. Messer nach einem der vorangehenden Ansprüche, dadurch gekennzeichnet, daß die Klemmlasche (13) auf ihrer Außenseite einen Betätigungsvorsprung (18) trägt, der materialeinheitlich mit der Klemmlasche (13) ausgebildet ist.

5. Messer nach einem der vorangehenden Ansprüche, dadurch gekennzeichnet, daß der Reibungsbeiwert zwischen der Klemmlasche (13) und der Klinge (12) größer ist als der Reibungsbeiwert zwischen der Klinge (12) und der Nutgrundfläche (23) des Griffes (11).

6. Messer nach Anspruch 5, dadurch gekennzeichnet, daß der Messergriff (11) aus Metall und die Klemmlasche (13) aus Kunststoff gefertigt sind.

## Claims

1. Knife (10) with a changeable blade (12), which is stopped at the end in an axial groove (15) at the end of the handle (11) between a plate (13) and the groove face (23), characterised in that the plate (13) is formed as a clamping plate (13) and is held slidably in longitudinal undercuts (16, 17) formed on either side along the base face (23) of the groove, which is formed so as to rise at its end remote from the cutting edge (22), so that the changeable blade (12) is stopped via a wedge thereby formed.

2. Knife according to claim 1, characterised in that the clamping plate (13) has a shoulder (20) oriented transversely to the longitudinal central axis (x) of the knife (10) and facing the base face (23) of the groove, against which shoulder (20) the rear end of the blade (12) bears.

3. Knife according to either of claims 1 or 2, characterised in that the blade (12) has an axial slot (21), which is open at its rear end and in which a corresponding, tongue-shaped projection (20) of the clamping plate (13), forming the shoulder, engages.

4. Knife according to one of the preceding claims, characterised in that the clamping plate (13) carries on its exterior an actuating projection (18), which is integral with the clamping plate (13).

5. Knife according to one of the preceding claims, characterised in that the friction correction value between the clamping plate (13) and the blade (12) is larger than the friction correction value between the blade (12) and the base face (23) of the groove of the handle (11).

6. Knife according to claim 5, characterised in that the knife handle (11) is made of metal and the clamping plate (13) of plastics material.

## Revendications

1. Couteau (10) muni d'une lame interchangeable (12) qui est bloquée en bout dans une rainure axiale (15) formée à l'extrémité du manche (11) entre un taquet (13) et la surface (23) de la rainure,
caractérisé en ce que le taquet (13) constitue un taquet coinceur (23) et est retenu mobile en translation par coulissement dans des contre-dépouilles longitudinales (16, 17) qui sont formées des deux côtés le long de la surface (23) du fond de la rainure, la surface (23) du fond de la rainure étant en pente montante vers son extrémité éloignée de l'arête tranchante (22), de sorte que la lame interchangeable (12) est bloquée par un coincement ainsi établi.

2. Couteau selon la revendication 1,
caractérisé en ce que le taquet coinceur (13) présente un épaulement (20) orienté transversalement à l'axe longitudinal (x) du couteau (10), dirigé vers la surface (23) du fond de la rainure, et contre lequel l'extrémité arrière de la lame (12) s'appuie.

3. Couteau selon une des revendications 1 et 2,
caractérisé en ce que la lame (12) présente une fente axiale (21) s'ouvrant vers son extrémité arrière, dans laquelle s'engage une saillie (20) correspondante, en forme de languette, qui forme l'épaulement du taquet coinceur (13).

4. Couteau selon une des revendications précédentes,
caractérisé en ce que le taquet coinceur (13) présente, sur sa surface extérieure, une saillie de manoeuvre (18) qui est venue de matière avec le taquet coinceur (13).

5. Couteau selon une des revendications précédentes,
caractérisé en ce que le coefficient de frottement entre le taquet coinceur (13) et la lame (12) est plus grand que le coefficient de frottement entre la lame (12) et la surface (23) du fond de rainure du manche (11).

6. Couteau selon la revendication 5, caractérisé en ce que le manche (11) du couteau est fabriqué en métal et que le taquet coinceur (13) est fabriqué en matière plastique.

# FIG.1

# FIG.2

# FIG.3

# FIG.4

# FIG.5

# FIG.6

# FIG.7

# FIG.8